(19) Europäisches Patentamt
European Patent Office
Office européen des brevets

(11) Veröffentlichungsnummer : **0 458 079 B1**

(12) # EUROPÄISCHE PATENTSCHRIFT

(45) Veröffentlichungstag der Patentschrift :
**20.07.94 Patentblatt 94/29**

(51) Int. Cl.$^5$ : **A61K 49/00,** C08G 69/10

(21) Anmeldenummer : **91106660.3**

(22) Anmeldetag : **25.04.91**

(54) **Ultraschall-Kontrastmittel, Verfahren zu ihrer Herstellung und Verwendung derselben als Diagnostika und Therapeutika.**

(30) Priorität : **26.04.90 DE 4013231**

(43) Veröffentlichungstag der Anmeldung :
**27.11.91 Patentblatt 91/48**

(45) Bekanntmachung des Hinweises auf die Patenterteilung :
**20.07.94 Patentblatt 94/29**

(84) Benannte Vertragsstaaten :
**AT BE CH DE DK ES FR GB IT LI NL**

(56) Entgegenhaltungen :
**EP-A- 0 123 235**
**EP-A- 0 224 934**
**EP-A- 0 274 127**
**EP-A- 0 327 490**
**WO-A-80/02365**

(73) Patentinhaber : **HOECHST AKTIENGESELLSCHAFT**
**D-65926 Frankfurt (DE)**

(72) Erfinder : **Erbel, Raimund, Prof. Dr.**
**Rieslingstrasse 36**
**W-6500 Mainz (DE)**
Erfinder : **Zotz, Rainer, Dr.**
**Salvatorstrasse 9**
**W-6500 Mainz (DE)**
Erfinder : **Krone, Volker, Dr.**
**Zu den Eichen 25**
**W-6238 Hofheim-Langenhain (DE)**
Erfinder : **Magerstädt, Michael, Dr.**
**Rheingaustrasse 19**
**W-6238 Hofheim am Taunus (DE)**
Erfinder : **Walch, Axel, Dr.**
**Hans-Sachs-Strasse 5**
**W-6000 Frankfurt am Main (DE)**

**EP 0 458 079 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft Ultraschall-Kontrastmittel, bestehend aus Mikropartikeln, welche ein Gas und Polyaminodicarbonsäure-co-imid-Derivate enthalten, Verfahren zu ihrer Herstellung und deren Verwendung als Diagnostika und Therapeutika.

Die Ultraschalldiagnostik hat in der Medizin wegen der komplikationslosen einfachen Handhabung sehr breite Anwendung gefunden. Ultraschallwellen werden an Grenzflächen von unterschiedlichen Gewebearten reflektiert. Die dabei entstehenden Echosignale werden elektronisch verstärkt und sichtbar gemacht.

Die Darstellung von Blutgefäßen und inneren Organen mittels Ultraschall erlaubt im allgemeinen nicht die Darstellung des darin vorhandenen Blutflusses. Flüssigkeiten, insbesondere Blut, liefern nur dann Ultraschallkontrast, wenn Dichteunterschiede zur Umgebung bestehen. Als Kontrastmittel werden in der medizinischen Ultraschalldiagnostik z.B. Gase enthaltende oder Gase produzierende Substanzen verwendet, da der Impedanzunterschied zwischen Gas und umgebendem Blut wesentlich größer ist, als der zwischen Flüssigkeiten oder Festkörpern und Blut (Levine R.A., J Am Coll Cardiol 3: 28, 1989; Machi I.J CU 11: 3, 1983).

In der Literatur sind mehrere Methoden zur Herstellung und Stabilisierung von Gasblasen bekannt. In dem US-Patent 4,276,885 wird die Herstellung von Gasbläschen mit definierter Größe beschrieben, die mit einer vor dem Zusammenfließen der Gasblasen schützenden Gelatinehülle umgeben sind. Die Aufbewahrung der fertigen Gasbläschen kann nur im eingefrorenen Zustand erfolgen, wobei die Gasbläschen zur Verwendung wieder auf Körpertemperatur gebracht werden müssen.

Die EP-A2-0 123 235 und 0 122 624 beschreiben Gase enthaltende Ultraschall-Kontrastmittel, die aus Mischungen von grenzflächenaktiven Substanzen mit einem Feststoff in einem flüssigen Träger bestehen. Die Ultraschall-Kontrastmittel werden durch einen aufwendigen Vermahlungsprozeß mit einer Luftstrahlmühle hergestellt. Die so hergestellten Partikel haben nur eine geringe Nutzungsdauer, weil sie die eingeschlossenen Gase schnell verlieren.

Die EP-A2-0 224 934 beschreibt Ultraschall-Kontrastmittel in Form von gasgefüllten Gelatine- oder Albuminhohlkörpern. Nachteilig ist jedoch die Verwendung von körperfremden oder denaturierten körpereigenen Proteinen wegen des damit verbundenen allergenen Risikos.

In der EP-A1-0 327 490 werden Mikropartikel beschrieben, die aus Amylosen oder synthetischen, bioabbaubaren Polymeren und einem Gas und/oder einer Flüssigkeit mit einem Siedepunkt von weniger als 60°C, bestehen. Nachteile dieser Polymere sind deren klebrige Konsistenz in Wasser oder Blut, deren schlechte Bioabbaubarkeit, deren Giftigkeit oder das Entstehen von toxischen Abbauprodukten.

Es wurde bereits vorgeschlagen (Deutsche Patentanmeldung P 40 02 736.8) Polyaminodicarbonsäure-co-imid-Derivate als bioabbaubare Depotzubereitungen von Arzneimitteln mit kontrollierter Wirkstoffabgabe einzusetzen.

Aufgabe der vorliegenden Erfindung war es Ultraschall-Kontrastmittel auf der Basis von Mikropartikeln zu entwickeln, die einen deutlichen Kontrast zum umgebenden Gewebe liefern, die so klein und stabil sind, daß sie ohne wesentlichen Gasverlust und im wesentlichen quantitativ die linke Herzhälfte nach intravenöser Applikation erreichen, gute Verträglickeit ohne allergenes Potential besitzen, nicht in Wasser oder Blut miteinander verklumpen und sich schnell und einfach herstellen lassen.

Es wurden Mikropartikel aus Polyaminodicarbonsäure-co-imid-Derivaten (Polydicarbonsäure-co-AHADS-Derivate) hergestellt, die sich überraschenderweise hervorragend als Ultraschall-Kontrastmittel eignen. Durch Einbau ungeöffneter Imidringe (AHADS-Ringe) ist insbesondere die Suspendierbarkeit der hergestellten Mikropartikel im Wasser hervorragend. Die Mikropartikel haben in wasserhaltigen Flüssigkeiten keine klebrige, schmierige Konsistenz und lagern sich kaum zusammen. Die Polymere bilden eine pharmakologisch inerte Matrix, in die das Gas eingeschlossen ist. In vivo werden diese Polymere zu untoxischen, nicht allergenen und nicht immunogenen Verbindungen metabolisiert und ausgeschieden. In Tierexperimenten konnte gezeigt werden, daß die Mikropartikel die Lunge im wesentlichen ohne signifikanten Gasverlust passieren und in beiden Herzhälften zu einem gleich intensiven Ultraschallkontrast führen. Die dabei aufgezeichneten Echokardiogramme zeigen keinerlei Wandbewegungsstörungen, während und bis zu 60 min nach der Applikation des Kontrastmittels. Ferner konnten sich keinerlei Veränderungen in einem sechskanaligen EKG, sowie in der mittels Tip Manometer ermittelten Kontraktilität, festgestellt werden.

Die erfindungsgemäßen Ultraschall-Kontrastmittel bewirken einen verbesserten Echogenitätsanstieg in Myokard und erlauben eine verbesserte Endokarddarstellung. Ferner können beispielswiese folgende Parameter besser beurteilt werden:

Kammergröße, Wandbewegungsstörungen, Schlagvolumen, Ejektionsfraktion oder intrakavitäre Massen, Z. B. Thromben oder Tumore. Ferner ermöglichen die erfindungsgemäßen Ultraschall-Kontrastmittel die Evaluierung von Fließmustern bei Klappeninsuffizienzen der linken und der rechten Herzhälfte, intrakardialen Shunts, sowie die verbesserte Darstellung der großen Gefäße bei kongenitalen Mißbildungen. Es wurde auch

eine massive Verstärkung des Dopplersignals beobachtet.

Die Erfindung betrifft somit Ultraschall-Kontrastmittel, bestehend aus Mikropartikeln, welche ein Gas und ein Polyaminodicarbonsäure-co-imid-Derivat der Formel I,

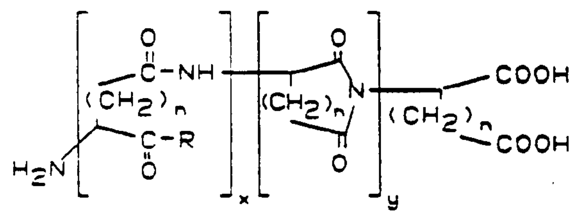

(I)

in der

| | |
|---|---|
| n | 1 oder 2 |
| x | 1 bis 500 |
| y | 1 bis 500 ist, wobei |
| x + y | 2 bis 1000 ist und |
| R | O-$R^1$ oder NH-$R^2$ bedeutet, worin |

| | |
|---|---|
| $R^2$ | H, $(CH_2)_m$-O$R^1$, $(CH_2)_m$-O-C(O)-$R^1$ oder $(CH_2)_m$-O-C(O)-O$R^1$ bedeutet und |
| m | 2 bis 6 ist und |
| $R^1$ | H, Aryl, Aralkyl, Arylalkenyl, Alkyl oder $C_3$-$C_8$-Cycloalkyl oder ein biologisch inaktiver Steroidalkohol oder eine Aminosäure bedeutet, wobei Aryl unsubstituiert ist oder substituiert ist mit $C_1$-$C_4$-Alkyl, $C_2$-$C_4$-Alkenyl, $C_1$-$C_4$-Alkylcarbonyloxy, $C_1$-$C_4$-Alkoxycarbonyl, $C_1$-$C_4$-Alkoxy oder Hydroxy |

wobei die für $R^1$ genannten Alkylreste 1 - 22 C-Atome und die Alkenylreste 2 - 22 C-Atome aufweisen, die nicht unterbrochen oder durch eine Carbonyloxy- oder Oxycarbonylgruppe unterbrochen sind, wobei die in eckige Klammern gesetzten Wiederholungseinheiten statistisch und/oder in Blöcken im Polymeren verteilt sind und wobei sowohl die mit x als auch die mit y gekennzeichneten Wiederholungseinheiten identisch oder unterschiedlich sind und wobei die Aminosäuren α- und/oder β-verknüpft sind, enthalten.

Unter Aryl werden aromatische Kohlenwasserstoffe verstanden wie Phenyl und Naphthyl, insbesondere Phenyl. Bei den angegebenen substituierten Arylresten sind 1 bis alle ersetzbaren Wasserstoffatome durch identische oder unterschiedliche Substituenten ersetzt. Bevorzugt sind die Arylreste mono- oder disubstituiert.

Die genannten Alkyl- und Alkenylreste können sowohl geradkettig als auch verzweigt sein.

Die biologisch inaktiven Steroidalkohole sind bevorzugt über ihre OH-Gruppe gebunden. Ein bevorzugter Steroidalkohol ist Cholesterol.

Bei den für $R^1$ genannten Aminosäuren handelt es sich bevorzugt um natürlich vorkommende Aminosäuren wie Tyr, Ala, Ser oder Cys, besonders bevorzugt um Tyr und Ala. Sie können sowohl über ihre $NH_2$- als auch über ihre COOH-Funktion gebunden sein.

Die Erfindung betrifft auch Verfahren zur Herstellung von Gas enthaltenden Mikropartikeln, die aus den obengenannten Polymeren bestehen oder diese enthalten und deren Verwendung auch in Abmischung mit anderen, bioverträglichen und/oder bioabbaubaren Polymeren oder physiologisch unbedenklichen Hilfsstoffen, für diagnostische oder therapeutische Verfahren.

Ein weiterer Gegenstand der Erfindung sind Diagnostika oder Therapeutika, bestehend aus mindestens einem der obengenannten Kontrastmittel.

Ferner betrifft die Erfindung auch Verfahren zur Herstellung von Diagnostika oder Therapeutika, die dadurch gekennzeichnet sind, daß man die obengenannten Ultraschall-Kontrastmittel mit einem physiologischen Träger und gegebenenfalls weiteren Zusatz- und/oder Hilfsstoffen in eine geeignete Darreichungsform bringt.

Im folgenden wird die Erfindung detailliert beschrieben.

Als Aminodicarbonsäuren werden Asparaginsäure und/oder Glutaminsäure eingesetzt, die in einer Polykondensationsreaktion zu den entsprechenden Polyimiden (Polyanhydroaminodicarbonsäuren, Formel II) reagieren. Durch partielle Umsetzung mit einer oder mehreren Verbindungen der Formeln III und/oder IV und/oder $NH_3$

$$HO\text{-}R^1 \qquad (III)$$

$$H_2N\text{-}(CH_2)_m\text{-}OH \qquad (IV),$$

worin m und $R^1$ wie oben zu Formel I definiert sind, erhält man einen α,β-Poly-D,L-Aminosäureester-co-imid der Formel VIII

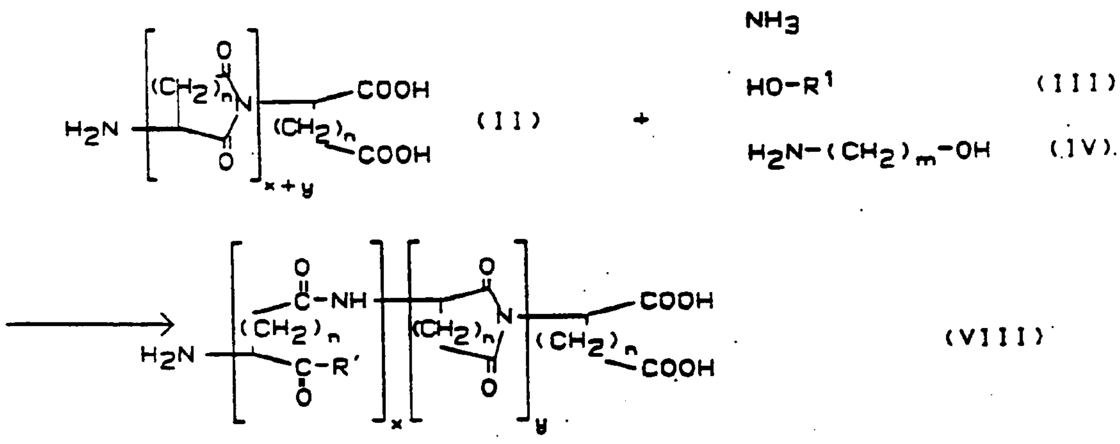

(II) (III) (IV) (VIII)

$R' = O-R^1,\ HN-(CH_2)_m-OH,\ NH_2$

Wesentlich bei dieser Umsetzung ist, daß die Polyanhydroaminodicarbonsäure (II) nur teilweise in die offenkettigen Derivate überführt wird. Der Anteil an ungeöffneten Anhydroaminodicarbonsäure-Einheiten beträgt 0,1 bis 99,9 %, bevorzugt 10 bis 90 %, (die Prozentangaben beziehen sich auf die Gesamtzahl der Wiederholungseinheiten im Gesamtpolymeren). In Abhängigkeit davon, nach welcher Seite der Imidring bei der oben beschriebenen Reaktion geöffnet wird, erhält man α- bzw. β-verknüpfte Aminosäuren. Bevorzugt eingesetzte Verbindungen der Formeln III und IV sind: 2-Aminoethanol, 3-Aminopropanol, 2-Aminopropanol, Alkohole mit 1 - 18 C-Atomen, insbesondere Methanol, Ethanol, Isoamylalkohol und Isopropylalkohol.

Ein Verfahren zur Herstellung von α,β-Poly-(2-hydroxyethyl)-DL-Aspartimid (PHEA) (Formel I; y = O; R = $NH-CH_2-CH_2-OH$) wird von P. Neri, G. Antoni, F. Benvenuti, F. Cocola, G. Gazzei, in J. Med. Chem. Bd. 16, 893 (1973) beschrieben. Eine allgemeine Arbeitsvorschrift zur Herstellung von PHEA findet sich in P. Neri, G. Antoni, Macromol. Synth. Bd. 8, 25. Auf diese Literaturstelle wird an dieser Stelle ausdrücklich Bezug genommen. Die Umsetzung erfolgt in hoher Ausbeute zu einem Produkt mit hohem Reinheitsgrad. In gleicher Weise lassen sich, durch unterstöchiometrischen Einsatz von $NH_3$ und/oder Verbindungen der Formeln III und/oder IV, die analogen Poly-asparaginsäurederivat-co-succinimid-Verbindungen der Formel VIII (n = 1) herstellen.

Zur Herstellung von reinem Poly-(hydroxyalkyl)-L-glutamin muß ein anderes, umständlicheres Verfahren angewendet werden, wie es in der US-PS 4 356 166 beschrieben ist. Dabei wird zunächst die γ-ständige COOH-Gruppe der L-Glutaminsäure durch Veresterung mit Benzylalkohol geschützt. Anschließend wird dieses γ-Benzyl-Glutamat mit Phosgen zu einem N-Carboxyanhydrid umgesetzt, welches dann nach Zugabe von Triethylamin in einem inerten Lösungsmittel polymerisiert, wobei man Poly-γ-(benzyl)-L-glutamat erhält. Die Abspaltung der Schutzgruppe erfolgt entweder durch Zugabe von einem HCl/HBr-Gemisch zur freien Poly-α-L-Glutaminsäure oder aber in Gegenwart von Hydroxyalkylaminen zu den analogen Poly-α-(hydroxyalkyl)-L-glutaminen. Eine allgemeine Arbeitsvorschrift zur Herstellung von Poly-α-(hydroxypropyl)-L-glutamin findet sich in der US-PS 4 356 166, auf die an dieser Stelle ausdrücklich Bezug genommen wird. In gleicher Weise lassen sich auch durch Einsatz von $NH_3$ und/oder Verbindungen der Formeln III und/oder IV die analogen Verbindungen der Formel VIII (n = 2) herstellen.

Gegenüber der aufwendigen Darstellung von reiner Polyglutaminsäure und deren Derivate kann man Glutaminsäure bis zu hohen Anteilen bei der einfachen Kondensation von Asparaginsäure mittels Phosphorsäure zu Polyanhydroasparaginsäure-co-glutaminsäure mit einbauen.

Die Polyaminosäureamid-co-imide der Formel VIII ($R' = HN-(CH_2)_m-OH$) können nun, sofern erforderlich, im folgenden Reaktionsschritt mit einer oder mehreren verschiedenen, biologisch inaktiven Verbindungen der Formel V und/oder VI und/oder VII

$$X-R^1 \quad (V)$$

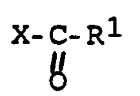 (VI) 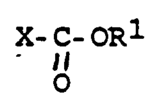 (VII)

zu weiteren Polyaminodicarbonsäure-co-AHADS-Derivaten umgesetzt werden. Hierbei steht X für eine Abgangsgruppe, die eine schonende Veresterung der Polymer-Alkoholgruppe ermöglicht. Bevorzugt sind Chlor,

Brom, Jod, Imidazolide, Anhydride oder Hydroxyl, insbesondere Chlor.

Die Umsetzung mit den Verbindungen des Formeltyps V, VI oder VII kann sowohl mit einer einzigen solchen Verbindung erfolgen als auch mit beliebigen Kombinationen dieser Verbindungen oder auch mit Verbindungen, die unterschiedliche, z.B. in der Art ihrer Verzweigung, insbesondere in ihrer Kettenlänge verschiedene Reste $R^1$ haben.

Die letztgenannte polymeranaloge Alkylierung bzw. Acylierung wird nach bekannten Verfahren der organischen Chemie durchgeführt. Sie verläuft selektiv an der Hydroxylfunktion (Formel VIII, R' = $HN-(CH_2)_m-OH$) zu Ethern, Estern bzw. Carbonaten, ohne weitere Funktionen am Ausgangspolymeren anzugreifen. Besonders eignet sich die Einhorn-Variante der Schotten-Baumann-Acylierung in Gegenwart von Pyridin. Dabei werden unter schonenden Bedingungen sehr hohe Derivatisierungsgrade (größer 70 %) erzielt.

Das Molekulargewicht der Polymeren beträgt 200 bis 100.000, bevorzugt 3.000 bis 70.000.

Verbindungen des Formeltyps V sind käuflich oder, sofern nicht, auf einfache Weise nach literaturbekannten Verfahren zu synthetisieren.

Die Chlorameisensäureester (Formel VII) erhält man durch Umsetzung von Phosgen mit den entsprechenden biologisch inaktiven, physiologisch unbedenklichen, aromatischen, araliphatischen, aliphatischen oder cycloaliphatischen, insbesondere unverzweigten Alkoholen. Besonders bevorzugt werden solche Alkohole eingesetzt, die eine geradzahlige Kohlenstoffatomanzahl aufweisen. Auf diese Weise erhält man auch die chlorformylierten Steroide. Prinzipiell sind somit alle biologisch inaktiven Steroide zugänglich, die reaktive Hydroxylgruppen aufweisen. Beispielsweise seien hier genannt: Cholesterol, Cholestanol, Coprostanol, Ergosterol, Sitosterol oder Stigmasterol.

Die ebenfalls einsetzbaren Säurechloride (Formel VI) erhält man beispielsweise aus den entsprechenden Carbonsäuren durch Umsetzung mit Phosphortrichlorid, Phosphorpentachlorid, Oxalylchlorid oder Thionylchlorid.

Verbindungen des Formeltyps V, VI oder VII, in denen eine Alkylkette durch eine Oxycarbonyl- oder Carbonyloxy-Gruppe unterbrochen ist, werden beispielsweise durch Umsetzung von cyclischen Dicarbonsäureanhydriden mit Alkoholen hergestellt. Die auf diese Weise erhaltenen Dicarbonsäuremonoester werden dann analog zu den oben beschriebenen Carbonsäuren, z.B. mit Oxalylchlorid zu den entsprechenden Säurechloriden umgesetzt.

Ein vorteilhaftes Verfahren zur Herstellung der Ultraschall-Kontrastmittel besteht darin, ein oder mehrere der Polyaminodicarbonsäure-co-imid-Derivate der Formel I in einem Lösungsmittel oder Lösungsmittelgemisch mit hohem Schmelzpunkt zu lösen oder diese Derivate mit einem oder mehreren weiteren Polymeren und/oder physiologisch unbedenklichen Hilfsstoffen zu mischen und in einem Lösungsmittel oder Lösungsmittelgemisch mit hohem Schmelzpunkt zu lösen und in ein kondensiertes kaltes Gas, z. B. flüssigen Stickstoff, zu vertropfen. Durch das Leidenfrostsche Phänomen entstehen dabei absolut runde Partikel. Als Lösungsmittel sind beispielsweise Alkohole, Dimethylformamid, Dimethylsulfoxid, Tetrahydrofuran, Methylenchlorid, Dioxan, Acetonitril oder Mischungen mit Alkoholen einsetzbar. Das hochschmelzende und mit Wasser mischbare Lösungsmittel wird z. B. durch Überführung der Mikropartikel in Wasser herausgelöst und das Polymere dabei ausgefällt, wobei die Kugelgestalt der Mikropartikel erhalten bleibt.

Besitzt das verwendete organische Lösungsmittel neben einem hohen Schmelzpunkt zugleich einen niedrigen Siedepunkt, so läßt sich dieses Vertropfungsverfahren weiter vereinfachen, indem das Lösungsmittel, z.B. tert. Butanol, direkt mittels Gefriertrocknung schonend entfernt werden kann.

Ein weiteres Verfahren zur Herstellung der Ultraschall-Kontrastmittel besteht darin, ein oder mehrere der Polyaminodicarbonsäure-co-imid-Derivate der Formel I in einem Lösungsmittel oder Lösungsmittelgemisch aufzulösen und gegebenenfalls nach Zusatz eines weiteren Lösungsmittel und/oder eines oder mehreren weiteren Polymeren auszufällen oder in Wasser zu dispergieren. Als weitere Polymere eignen sich beispielsweise Polyvinylalkohol (®Mowiol 28-99) oder Polyoxyethylenpolyoxypropylen (®Pluronic F 127). Als weiteres Lösungsmittel können z. B. Ether verwendet werden. Mikropartikel mit einem Durchmesser von 0,5 bis 15 μm erhält man durch starkes Rühren z. B. mit einem Mixer (25000 UPM). Anschließend werden die Lösungsmittel z. B. durch lyophilisieren entfernt.

Ein besonders vorteilhaftes Verfahren besteht darin, die Mikropartikel durch Sprühtrocknung zu gewinnen. Dazu werden ein oder mehrere Polyaminodicarbonsäure-co-imid-Derivate der Formel I gelöst oder diese Derivate werden mit einem oder mehreren weiteren Polymeren und/oder physiologisch unbedenklichen Hilfsstoffen gemischt und in Lösung gebracht. Als Lösungsmittel oder Lösungsmittelgemische eignen sich beispielsweise Alkohol, Dimethylformamid, Dimethylsulfoxid, Tetrahydrofuran, Methylenchlorid, Dioxan oder Acetonitril. Anschließend wird die Lösung in einem Sprühtrockner zu Mikropartikeln versprüht.

Bei dem beschriebenen Verfahren können die Polymere der Formel I allein oder auch als Gemisch verschiedener Polymere der Formel I verwendet werden. Diese Polymere können auch in Mischungen mit anderen bioabbaubaren und/oder bioverträglichen Polymeren (z. B. ®Pluronic F68, PHEA, Dextrane, Polyethylenglykole, Hydroxyethylstärke und andere abbaubare oder ausscheidbare Polysaccharide) oder physiologisch unbedenklichen Hilfsstoffen ( z. B. Polymerweichmacher) eingesetzt werden.

Die Mikropartikel enthalten Gas beispielsweise Luft, Stickstoff, Edelgase wie Helium, Neon, Argon oder Krypton, Wasserstoff, Kohlendioxid, Sauerstoff, oder deren Gemische. Die Mikropartikel werden mit einem Gas beladen, indem beispielsweise die Mikropartikel nach der Lyophilisierung in einer entsprechenden Gasatmosphäre gelagert oder bei der Sprühtrocknung direkt bei der Herstellung in einer entsprechenden Gasatmosphäre gewonnen werden.

Die erfindungsgemäßen Ultraschall-Kontrastmittel werden vor Applikation durch Zusatz von einem oder mehreren physiologisch annehmbaren Trägern und gegebenenfalls weiteren Zusatz- und/oder Hilfsstoffen in eine geeignete diagnostische oder therapeutische Darreichungsform überführt. Die Ultraschall-Kontrastmittel werden beispielsweise vor Applikation durch Zusatz von Wasser und Mischen suspendiert.

Durch Zugabe osmotisch aktiver Substanzen, beispielsweise Kochsalz, Galaktose, Glukose, Fruktose, kann die physiologische Isotonie der Partikelsuspension hergestellt werden.

Bei den beschriebenen Verfahren zur Herstellung der erfindungsgemäßen Ultraschall-Kontrastmittel kann man Partikelgrößen erreichen bei denen 90% der Partikel zwischen 0,1 μm und 15 μm liegen. Mit dem Sprühtrocknungsverfahren können Partikelgrößenverteilungen erreicht werden, bei denen 90 % der Partikel kleiner als **3** μm sind. Größere Teilchen werden durch Aussieben, beispielsweise mit einem 15 μm Siebgewebe und/oder 3 μm Siebgewebe entfernt. Bei der Verwendung dieser Mikropartikel als Ultraschall-Kontrastmittel zur Diagnose von Herz-Kreislauf-Erkrankungen haben sich Partikelgrößen von 0,1 μm bis 7 μm bewährt, vorteilhaft werden Partikelgrößen von 0,1 μm bis 3 μm eingesetzt. Die Ultraschall-Kontrastmittel werden beispielsweise in die Blutbahn injiziert. Je Injektion werden 0,1 mg bis 1000 mg der Mikropartikel, bevorzugt 1 mg bis 100 mg, eingesetzt.

Die im vorstehenden beschriebenen Ultraschall-Kontrastmittel können sowohl für diagnostische als auch therapeutische Verfahren verwendet werden. Die Verwendung der erfindungsgemäßen Ultraschall-Kontrastmittel ist nicht nur auf die Sichtbarmachung des Blutstroms im rechtsventrikulären Teil des Blutkreislaufes nach venöser Applikation beschränkt. Die Ultraschall-Kontrastmittel können mit ausgezeichnetem Erfolg für die Untersuchung der linken Herzseite und des Myocards verwendet werden. Ferner ist es auch möglich andere vom Blut versorgte Organe wie Leber, Milz, Niere oder Gehirn mit diesen Kontrastmitteln sichtbar zu machen.

Die erfindungsgemäßen Ultraschall-Kontrastmittel eignen sich aber auch zun Sichtbarmachen von Hohlräumen in Menschen, Tieren oder Pflanzen, beispielsweise Harnblase, Harnleiter, Gebärmutter oder Vagina.

In den folgenden Beispielen wird die Erfindung im einzelnen beschrieben. Prozentangaben beziehen sich auf das Gewicht, sofern nicht anders angegeben.

**Beispiel 1**

Herstellung von Polysuccinimid-co-α,β-(hydroxy-ethyl)-D,L-aspartamid (70:30)

10 g (103 mmol) Polyanhydroasparaginsäure werden in etwa 40 ml N,N-Dimethylformamid (DMF) gegebenenfalls unter vorsichtigen Erwärmen gelöst. Zu dieser Lösung werden 1,83 g (30 mmol) frisch destilliertes 2-Aminoethanol zugetropft und bei Raumtemperatur über Nacht gerührt. Der Reaktionsansatz wird in Butanol gefällt und mit getrocknetem Aceton mehrfach gewaschen. Die Trocknung erfolgt im Vakuum bei erhöhter Temperatur. Das weiße, wasserlösliche Produkt entsteht zu annähernd 100 % und wird NMR-spektroskopisch auf Rückstände von DMF und Butanol geprüft. Das eingesetzte molare Verhältnis von Polyanhydroasparaginsäure zu Aminoethanol entspricht etwa der Copolymerenzusammensetzung.

**Beispiel 2**

Herstellung von 4-Chlor-4-oxobuttersäure-n-butylester

Bernsteinsäuremonobutylester wird mit überschüssigem Thionylchlorid und einem Tropfen DMF versetzt. Die Reaktion erfolgt unter Gasentwicklung. Man läßt den Ansatz über Nacht unter Feuchtigkeitsausschluß rühren und destilliert anschließend das überschüssige Thionylchlorid bei Normaldruck ab. Das zurückbleibende Rohprodukt wird bei 0,05 mbar fraktioniert destilliert und das Reinprodukt bei ca. 70°C gewonnen. Bei der IR-spektroskopischen Charakterisierung weist das Produkt Banden bei 1800 $cm^{-1}$ (Säurechlorid) und 1740 $cm^{-1}$ (Ester) von gleicher Intensität auf.

**Beispiel 3**

Herstellung von Polysuccinimid-co-α,β-(butyloxycarbonylpropionyloxyethyl)-D,L-aspartamid (70:30)

6 g Polysuccinimid-co-α,β-(hydroxyethyl)-D,L-aspartamid (= 16 mmol Hydroxyethylgruppen), dargestellt wie in Beispiel 1 beschrieben, werden in 100 ml trockenem N,N-Dimethylformamid (DMF) gelöst. Nach Zugabe von 4 g (50 mmol) Pyridin wird auf 0°C gekühlt und unter Rühren innerhalb 15 Minuten 4,8 g (25 mmol) 4-Chlor-4-oxobuttersäure-n-butylester (siehe Bsp. 2) zugegeben. Der Ansatz wird über Nacht gerührt und in 0,5 l Ether gefällt. Das ausgefallene Produkt wird abgesaugt, mit Ether, Aceton, Wasser, Aceton und Ether gewaschen. Man erhält etwa 8 g eines weißen Polymeren mit einem Substitutionsgrad von annähernd 100 % (NMR-spektroskopisch überprüfbar). Das entstandene Polymer ist z. B. in Acetonitril mit einer Spur Dimethylsulfoxid (DMSO), in DMSO oder DMF löslich.

**Beispiel 4**

Herstellung von Polysuccinimid-co-α,β-(nonylcarbonyloxyethyl)-D,L-aspartamid (50:50)

6 g eines Polysuccinimid-co-α,β-(hydroxyethyl)-D,L-aspartamids (50:50) (≙ 24 mmol Hydroxyethylgruppen), das analog Beispiel 1 aus Polyanhydroasparaginsäure (MG = 14000) und 2-Aminoethanol (molares Verhältnis 2:1) hergestellt wurde, werden in 100 ml trockenem DMF gelöst, mit 8 g (100 mmol) trockenem Pyridin versetzt und auf 0°C gekühlt. Es werden langsam 9,6 g destilliertes Decansäurechlorid zugetropft und analog Beispiel 3 weitergearbeitet. Man erhält etwa 8 g eines weißen, vollständig substituierten Polymers (NMR-Kontrolle), das z. B. in Dichlormethan und THF mit jeweils einer Spur DMSO oder in Methanol/Dichlormethan-Gemischen löslich ist.

**Beispiel 5**

Herstellung von Polysuccinimid-co-α,β-(nonylcarbonyloxyethyl)-D,L-aspartamid verschiedener Copolymerenzusammensetzung und unterschiedlichen Molekulargewichtes

Analog Beispiel 1 wurden verschiedene Polysuccinimid-co-α,β-(hydroxyethyl)-D,L-aspartamide u. a. der Zusammensetzung 70:30, 50:50 und 30:70 aus Polyanhydroasparaginsäuren unterschiedlichen Molekulargewichtes (MG = 7000; ca. 13000; 30000) hergestellt und mit Decansäurechlorid, wie in Beispiel 4 beschrieben zu den entsprechenden Polysuccinimid-co-α,β-(nonylcarbonyloxyethyl)-D,L-aspartamiden umgesetzt.

a) - Polysuccinimid-co-α,β-(nonylcarbonyloxy-ethyl)-D,L-aspartamid (70:30) aus Polyanhydroasparaginsäure (MG = 7000); charakterisiert durch NMR

b) - Polysuccinimid-co-α,β-(nonylcarbonyloxy-ethyl)-D,L-aspartamid (70:30) aus Polyanhydroasparaginsäure (MG = 14000); charakterisiert durch NMR

c) - Polysuccinimid-co-α,β-(nonylcarbonyloxy-ethyl)-D,L-aspartamid (70:30) aus Polyanhydroasparaginsäure (MG = 30000); charakterisiert durch NMR

d) - Polysuccinimid-co-α,β-(nonylcarbonyloxy-ethyl)-D,L-aspartamid (30:70) aus Polyanhydroasparaginsäure (MG = 12000); charakterisiert durch NMR

**Beispiel 6**

Herstellung von Polysuccinimid-co-α,β-(octyloxycarbonyloxyethyl)-D,L-aspartamid (70:30)

6 g Polysuccinimid-co-α,β-(hydroxyethyl)-D,L-aspartamid (70:30) (≙ 16 mmol Hydroxyethylgruppen), dargestellt wie in Beispiel 1 beschrieben aus Polyanhydroasparaginsäure (MG = 37000) und Aminoethanol, werden analog Beispiel 3 mit 4,8 g (25 mmol) Octylchloroformiat umgesetzt sowie entsprechend aufgearbeitet. Man erhält etwa 8 g eines weißen, vollständig substituierten Polymers, das in THF oder Methanol/ Dichlormethan-Gemischen löslich ist.

**Beispiel 7**

Herstellung von Polysuccinimid-co-α,β-(nonylcarbonyloxyethyl)-co-α,β-(hydroxyethyl)-D,L-aspartamid (60:20:20)

6 g Polysuccinimid-co-α,β-(hydroxyethyl)-D,L-aspartamid (60:40) (≙ 20 mmol Hydroxyethylgruppen), das analog Beispiel 1 aus Polyanhydroasparaginsäure und 2-Aminoethanol (molares Verhältnis 6:4) hergestellt wurde, werden analog Beispiel 3 mit 2,3 g Decansäurechlorid (≙ 12 mmol) zur Reaktion gebracht. Auf Grund des unvollständigen Umsatzes (verhältnismäßig geringer Überschuß an Säurechlorid) werden die freien OH-Gruppen nur zur Hälfte verestert. Es entstehen etwa 7 g eines weißen Polymeren. Mikropartikel dieser Substanz zeigen eine feste Konsistenz in Wasser und sind gut suspendierbar.

**Beispiel 8**

Herstellung von Polysuccinimid-co-α,β-(oleyl-oxyethyl)-D,L-aspartamid (10:90)

6 g Polysuccinimid-co-α,β-(hydroxyethyl)-D,L-aspartamid (10:90) (≙ 40 mmol Hydroxyethylgruppen), dargestellt analog Beispiel 1 mit einem molaren Verhältnis von Polyanhydroasparaginsäure zu 2-Aminoethanol wie 1:9, werden mit 20 g destilliertem Ölsäurechlorid analog Beispiel 3 umgesetzt. Die heterogene Reaktionsmischung wird durch Zugabe von Dichlormethan homogen. Es wird zweifach in Methanol, das auf -20°C gekühlt ist, gefällt. Das gelblich gefärbte Polymer ist thermoplastisch.

**Beispiel 9**

a) Herstellung von Mikropartikeln

40 mg Polysuccinimid-co-α,β-(nonylcarbonyloxy-ethyl)-D,L-aspartamid (50:50) aus Beispiel 4 werden in 1 ml Methylenchlorid/Methanol (Volumenanteil 50/1) gelöst. Die Lösung wird unter Rühren (800 Upm) in ein Becherglas mit 60 ml 0,1 Gew.-%iger, wäßriger Polyvinylalkohol-Lösung (®Mowiol 28-99) eingebracht, die mit 0,3 ml Methylenchlorid/Methanol (50/1) gesättigt ist. Gleichzeitig wird die Lösung mit einem Mixer (25000 UPM) fein dispergiert.

Nach 5 Minuten wird der Inhalt in ein Becherglas mit 200 ml Wasser gegeben und 30 Minuten gerührt (200 Upm). Das überstehende Wasser wird abdekantiert und die Mikropartikel werden lyophilisiert (Durchmesser nach Lyophilisation: 0,5 bis 15 μm).

**Beispiel 10**

Herstellung von Mikropartikeln

80 mg Polysuccinimid-co-α,β-(octylcarbonyloxy-ethyl)-D,L-aspartamid (70:30) aus Beispiel 6 werden bei 50°C in 1 ml Dimethylsulfoxid gelöst und mit 20 mg Hydroxypropylcellulose (®Klucel M.) versetzt. Die Lösung der beiden Polymeren wird mit einer Kanüle (Einwegspritze, Kanülendurchmesser außen 0,6 mm) in eine Vorlage von flüssigem Stickstoff (100 ml) eingetropft.

Die entstandenen Mikropartikel werden in 200 ml Wasser überführt und 2 Stunden von restlichem Lösungsmittel extrahiert. Überschüssiges Wasser wird abdekantiert und die Mikropartikel werden lyophilisiert (Durchmesser nach Lyophilisation: 1-2 μm).

**Beispiel 11**

Herstellung von Mikropartikeln

Je 4 g von Polysuccinimid-co-α,β-(octyloxycarbonyloxyethyl)-D,L-aspartamid (A) (Beispiel 6) und Polysuccinimid-co-α,β-(nonylcarbonyloxyethyl)-D,L-aspartamid (B) (Beispiel 5 d) werden in den in Tabelle 1 angegebenen Lösungsmitteln zu 2 % gelöst. Anschließend werden die Polymere in einem Sprühtrockner (Mini Spray Dryer Büchi 190, Firma Büchi, W-Germany) zu Mikropartikeln versprüht.

**Tabelle 1:**

| Substanz | Lösungsmittel | Partikelgröße 10% kleiner | 50% kleiner | 90% kleiner |
|---|---|---|---|---|
| A | THF | 1,6 µm | 3,6 µm | 6,7 µm |
| B | $CH_2Cl_2$ | 2,4 µm | 3,6 µm | 6,7 µm |
| B | $CH_2Cl_2$/ Methanol 3,6:1 (Vol.) | 1,3 µm | 1,9 µm | 3,0 µm |
| B | $CH_2Cl_2$/ Methanol 2:3 (Vol.) | 1,2 µm | 1,8 µm | 2,6 µm |
| B | THF/Methanol 3,6:1 (Vol.) | 1,3 µm | 1,9 µm | 2,7 µm |

Die Größenverteilung der Mikropartikeln ist in einem Cilas-Granulometer 715 bestimmt worden.

Jeweils 30 mg Portionen der oben hergestellten Mikropartikel werden in 1,5 ml Suspensionshilfsmittel dispergiert. Die Suspensionshilfsmittel bestehen aus 150 mg Dextran 40 (Firma Roth, W.-Germany), 7,5 mg Polysorbat und 13,5 mg NaCl in 1,5 ml destilliertem Wasser. Die Suspensionen werden mit Siebgeweben (15 µm und 3 µm Maschenweite) filtriert und anschließend lyophilisiert. Vor der Applikation werden die Mikropartikel mit Wasser suspendiert.

**Beispiel 12**

Echokardiographische Untersuchung am Hund

30 mg Mikropartikel (hergestellt nach Beispiel 11, Substanz B, $CH_2Cl_2$/Methanol 2:3 (Vol.)) Werden in 1,5 ml destilliertem Wasser mit Hilfe eines Glasstabes resuspendiert. Mit einer Injektionsspritze wird diese Suspension in eine periphere Vene injiziert. Ein Ultraschallkopf eines Ultraschallgerätes (Toshiba, FSH 160a, Japan) wird an den Thorax des Versuchstieres gehalten, so daß ein typischer Querschnitt durch das rechte und linke Herz erhalten wird. Sobald das Ultraschall-Kontrastmittel die rechte Herzhälfte erreicht, kann man auf dem Monitor des Ultraschallgerätes erkennen, wie das durch das Kontrastmittel markierte Blut den rechten Vorhof erreicht, dann den rechten Ventrikel und anschließend das Herz über die Pulmonalarterie wieder verläßt. Nach der Lungenpassage wird die linke Herzhälfte durch das Kontrastmittel erkennbar. Der Ultraschallkontrast vor und nach der Lungenpassage ist von gleicher Intensität, so daß man von einem im wesentlichen vollständigen Verbleib der Luft in den Polymeren sowie im wesentlichen verlustfreien Transport der Mikropartikel zur linken Herzhälfte ausgehen kann.

## Patentansprüche

### Patentansprüche für folgende Vertragsstaaten : AT, BE, CH, DE, DK, FR, GB, IT, LI, NL

1. Ultraschall-Kontrastmittel, bestehend aus Mikropartikeln, welche ein Gas und ein Polyaminodicarbonsäure-co-imid-Derivat der Formel I,

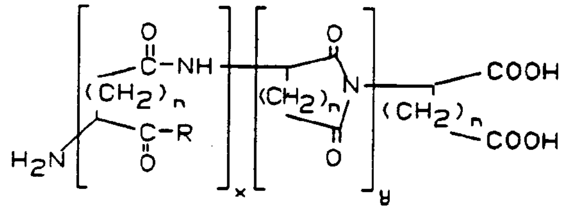

(I)

in der

n 1 oder 2
x 1 bis 500
y 1 bis 500 ist, wobei
x + y 2 bis 1000 ist und
R O-$R^1$ oder NH-$R^2$ bedeutet, worin
$R^2$ H, $(CH_2)_m$-$OR^1$, $(CH_2)_m$-O-C(O)-$R^1$ oder $(CH_2)_m$-O-C(O)-$OR^1$ bedeutet und
m 2 bis 6 ist und
$R^1$ H, Aryl, Aralkyl, Arylalkenyl, Alkyl oder $C_3$-$C_8$-Cycloalkyl oder ein biologisch inaktiver Steroidalkohol oder eine Aminosäure bedeutet, wobei Aryl unsubstituiert ist oder substituiert ist mit $C_1$-$C_4$-Alkyl, $C_2$-$C_4$-Alkenyl, $C_1$-$C_4$-Alkylcarbonyloxy, $C_1$-$C_4$-Alkoxycarbonyl, $C_1$-$C_4$-Alkoxy oder Hydroxy

wobei die für $R^1$ genannten Alkylreste 1 - 22 C-Atome und die Alkenylreste 2 - 22 C-Atome aufweisen, die nicht unterbrochen oder durch eine Carbonyloxy- oder Oxycarbonylgruppe unterbrochen sind, wobei die in eckige Klammern gesetzten Wiederholungseinheiten statistisch und/oder in Blöcken im Polymeren verteilt sind und wobei sowohl die mit x als auch die mit y gekennzeichneten Wiederholungseinheiten identisch oder unterschiedlich sind und wobei die Aminosäuren $\alpha$- und/oder $\beta$-sind, enthalten.

2. Ultraschall-Kontrastmittel nach Anspruch 1, worin in Formel I,
R NH-$R^2$ bedeutet und
m 2 ist und
$R^1$ H, Aryl, Aralkyl, Alkyl oder $C_5$-$C_6$-Cycloalkyl
bedeutet, wobei die Alkylreste 1 - 22 C-Atome aufweisen.

3. Ultraschall-Kontrastmittel nach Anspruch 1, worin in Formel I,
R O-$R^1$ bedeutet und
$R^1$ Aryl, Aralkyl, Alkyl oder $C_5$-$C_6$-Cycloalkyl bedeutet,
wobei die Alkylreste 1 - 22 C-Atome aufweisen.

4. Ultraschall-Kontrastmittel nach einem oder mehreren der Ansprüche 1 bis 3, dadurch gekennzeichnet, daß die Mikropartikel als Gas, Luft, Stickstoff, Edelgase, Wasserstoff, Kohlendioxid, Sauerstoff oder Mischungen dieser Gase enthalten.

5. Verfahren zur Herstellung eines Ultraschall-Kontrastmittels nach Anspruch 1, dadurch gekennzeichnet,
a) daß man eine Lösung eines oder mehrerer Polyaminodicarbonsäure-co-imid-Derivate der Formel I oder eine Lösung dieses oder dieser Derivate, die zusätzlich ein oder mehrere weitere Polymere und/oder physiologisch unbedenklichen Hilfsstoffe enthält, sprühtrocknet, oder daß man
b) ein oder mehrere Polyaminodicarbonsäure-co-imid-Derivate der Formel I in einem Lösungsmittel oder Lösungsmittelgemisch mit hohem Schmelzpunkt löst oder diese Derivate mit einem oder mehreren weiteren Polymeren und/oder physiologisch unbedenklichen Hilfsstoffen mischt und in einem Lösungsmittelgemisch mit hohem Schmelzpunkt löst und dann in ein kondensiertes kaltes Gas tropft und anschließend das Lösungsmittel entfernt, oder daß man
c) ein oder mehrere Polyaminodicarbonsäure-co-imid-Derivate der Formel I in einem Lösungsmittel

oder Lösungsmittelgemisch löst und anschließend gegebenenfalls nach Zusatz eines weiteren Lösungsmittels und/oder eines oder mehreren weiteren Polymeren ausfällt oder in Wasser dispergiert und die erhaltene Suspension von Lösungsmitteln befreit.

6. Verwendung von Ultraschall-Kontrastmittel nach einem oder mehreren der Ansprüche 1 bis 4 zur Herstellung von Diagnostika oder Therapeutika.

7. Verwendung von Ultraschall-Kontrastmittel nach einem oder mehreren der Ansprüche 1 bis 4 zur Untersuchung von Hohlräumen in Menschen, Tieren oder Pflanzen.

8. Verwendung von Ultraschall-Kontrastmittel nach einem oder mehreren der Ansprüche 1 bis 4 zur Diagnose von Herz-Kreislauf-Erkrankungen.

9. Diagnostikum oder Therapeutikum, bestehend aus mindestens einem Ultraschall-Kontrastmittel nach einem oder mehreren der Ansprüche 1 bis 4 oder mindestens einem nach dem Verfahren 5 hergestellten Ultraschall-Kontrastmittel.

10. Verfahren zur Herstellung eines Diagnostikums oder Therapeutikums gemäß Anspruch 9, dadurch gekennzeichnet, daß man mindestens ein Ultraschall-Kontrastmittel gemäß einem oder mehreren der Ansprüche 1 bis 4 mit einen physiologischen Träger und gegebenenfalls weiteren Zusatz- und/oder Hilfsstoffen in eine geeignete Darreichungsform bringt.

**Patentansprüche für folgenden Vertragsstaat : ES**

1. Verfahren zur Herstellung eines Ultraschall-Kontrastmittels, bestehend aus Mikropartikeln, welche ein Gas und ein Polyaminodicarbonsäure-co-imid-Derivat der Formel I,

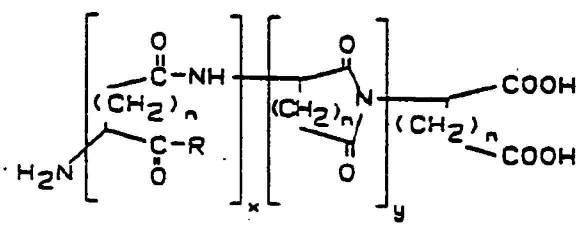

(I)

in der

| | |
|---|---|
| n | 1 oder 2 |
| x | 1 bis 500 |
| y | 1 bis 500 ist, wobei |
| x + y | 2 bis 1000 ist und |
| R | O-$R^1$ oder NH-$R^2$ bedeutet, worin |

- $R^2$ H, $(CH_2)_m$-O$R^1$, $(CH_2)_m$-O-C(O)-$R^1$ oder $(CH_2)_m$-O-C(O)-O$R^1$ bedeutet und
- m 2 bis 6 ist und
- $R^1$ H, Aryl, Aralkyl, Arylalkenyl, Alkyl oder $C_3$-$C_8$-Cycloalkyl oder ein biologisch inaktiver Steroidalkohol oder eine Aminosäure bedeutet, wobei Aryl unsubstituiert ist oder substituiertist mit $C_1$-$C_4$-Alkyl, $C_2$-$C_4$-Alkenyl, $C_1$-$C_4$-Alkylcarbonyloxy, $C_1$-$C_4$-Alkoxycarbonyl, $C_1$-$C_4$-Alkoxy oder Hydroxy

wobei die für $R^1$ genannten Alkylreste 1 - 22 C-Atome und die Alkenylreste 2 - 22 C-Atome aufweisen, die nicht unterbrochen oder durch eine Carbonyloxy- oder Oxycarbonylgruppe unterbrochen sind, wobei die in eckige Klammern gesetzten Wiederholungseinheiten statistisch und/oder in Blöcken im Polymeren verteilt sind und wobei sowohl die mit x als auch die mit y gekennzeichneten Wiederholungseinheiten identisch oder unterschiedlich sind und wobei die Aminosäuren α- und/oder β-verknüpft sind, enthalten, dadurch gekennzeichnet, daß man

a) eine Lösung eines oder mehrerer Polyaminodicarbonsäure-co-imid-Derivate der Formel I oder eine Lösung dieses oder dieser Derivate, die zusätzlich ein oder mehrere weitere Polymere und/oder physiologisch unbedenklichen Rilfsstoffe enthält, sprühtrocknet, oder daß man

b) ein oder mehrere Polyaminodicarbonsäure-co-imid-Derivate der Formel I in einem Lösungsmittel oder Lösungsmittelgemisch mit hohem Schmelzpunkt löst oder diese Derivate mit einem oder mehreren

weiteren Polymeren und/oder physiologisch unbedenklichen Hilfsstoffen mischt und in einem Lösungsmittel oder Lösungsmittelgemisch mit hohem Schmelzpunkt löst und dann in ein kondensiertes kaltes Gas tropft und anschließend das Lösungsmittel entfernt, oder daß man

c) ein oder mehrere Polvaminodicarbonsäure-co-imid-Derivate der Formel I in einem Lösungsmittel oder Lösungsmittelgemisch löst und anschließend gegebenenfalls nach Zusatz eines weiteren Lösungsmittels und/oder eines oder mehreren weiteren Polymeren ausfällt oder in Wasser dispergiert und die erhaltene Suspension von Lösungsmitteln befreit.

2. Verfahren nach Anspruch 1, dadurch gekennzeichnet, daß man Verbindungen der Formel I, worin

| | |
|---|---|
| R | NH-$R^2$ bedeutet und |
| m | 2 ist und |
| $R^1$ | H, Aryl, Aralkyl, Alkyl oder $C_5$-$C_6$-Cycloalkyl |

bedeutet, wobei die Alkylreste 1 - 22 C-Atome aufweisen, herstellt.

3. Verfahren nach Anspruch 1 oder 2, dadurch gekennzeichnet, daß man Verbindungen der Formel I, worin

| | |
|---|---|
| R | O-$R^1$ bedeutet und |
| $R^1$ | Aryl, Aralkyl, Alkyl oder $C_5$-$C_6$-Cycloalkyl bedeutet, |

wobei die Alkylreste 1-22 C-Atome aufweisen, herstellt.

4. Verfahren nach einem oder mehreren der Ansprüche 1 bis 3, dadurch gekennzeichnet, daß man Mikropartikel herstellt, die als Gas, Luft, Stickstoff, Edelgase, Wasserstoff, Kohlendioxid, Sauerstoff oder Mischungen dieser Gase enthalten.

5. Verwendung von Ultraschall-Kontrastmitteln, erhalten nach einem oder mehreren der Ansprüche 1 bis 4, zur Herstellung von Diagnostika oder Therapeutika.

6. Verwendung von Ultraschall-Kontrastmitteln, erhalten nach einem oder mehreren der Ansprüche 1 bis 4, zur Herstellung von Diagnostika zur Untersuchung von Hohlräumen in Menschen, Tieren oder Pflanzen.

7. Verwendung von Ultraschall-Konstrastmitteln nach einem oder mehreren der Ansprüche 1 bis 4 zur Herstellung von Diagnostika zur Diagnose von Herz-Kreislauf-Erkrankungen.

8. Verfahren zur Herstellung eines Diagnostikums oder Therapeutikums, dadurch gekennzeichnet, daß man mindestens ein Ultraschall-Kontrastmitel erhalten nach einem oder mehreren der Ansprüche 1 bis 4 mit einem physiologischen Träger und gegebenenfalls weiteren Zusatz- und/oder Hilfsstoffen in eine geeignete. Darreichungsform bringt.

## Claims

**Claims for the following Contracting States : AT, BE, CH, DE, DK, FR, GB, IT, LI, NL**

1. An ultrasonic contrast agent composed of micro-particles which contain a gas and a polyamino-dicarboxylic acid-co-imide derivative of the formula I,

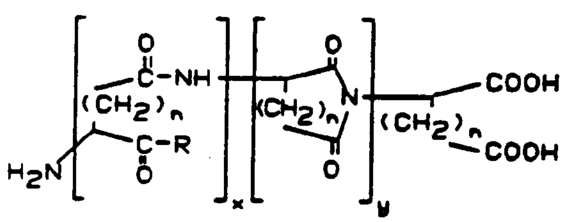

(I)

in which

| | |
|---|---|
| n | is 1 or 2 |
| x | is 1 to 500 |
| y | is 1 to 500, where |
| x + y | is 2 to 1000, and |

R is O-$R^1$ or NH-$R^2$, in which

$R^2$ is H, $(CH_2)_m$-O$R^1$, $(CH_2)_m$-O-C(O)-$R^1$ or $(CH_2)_m$-O-C(O)-O$R^1$, and

m is 2 to 6, and

$R^1$ is H, aryl, aralkyl, arylalkenyl, alkyl or $C_3$-$C_8$-cycloalkyl or a biologically inactive steroid alcohol or an amino acid, where aryl is unsubstituted or is substituted by $C_1$-$C_4$-alkyl, $C_2$-$C_4$-alkenyl, $C_1$-$C_4$-alkylcarbonyloxy, $C_1$-$C_4$- alkoxycarbonyl, $C_1$-$C_4$-alkoxy or hydroxyl,

where the alkyl radicals specified for $R^1$ have 1-22 carbon atoms and the alkenyl radicals have 2-22 carbon atoms, which are not interrupted or are interrupted by a carbonyloxy or oxycarbonyl group, where the repeating units placed in square brackets are distributed randomly and/or in blocks in the polymer, and where both the repeating units labeled with x and those labeled with y are identical or different and where the amino acids are α- and/or β-linked.

**2.** An ultrasonic contrast agent as claimed in claim 1, wherein in formula I

R is NH-$R^2$ and

m is 2 and

$R^1$ is H, aryl, aralkyl, alkyl or $C_5$-$C_6$-cycloalkyl, where the alkyl radicals have 1-22 carbon atoms.

**3.** An ultrasonic contrast agent as claimed in claim 1, wherein in formula I

R is O-$R^1$ and

$R^1$ is aryl, aralkyl, alkyl or $C_5$-$C_6$-cycloalkyl, where the alkyl radicals have 1-22 carbon atoms.

**4.** An ultrasonic contrast agent as claimed in one or more of claims 1 to 3, wherein the microparticles contain air, nitrogen, noble gases, hydrogen, carbon dioxide, oxygen or mixtures of these gases as gas.

**5.** A process for the preparation of an ultrasonic contrast agent as claimed in claim 1, which comprises

a) a solution of one or more polyamino-dicarboxylic acid-co-imide derivatives of the formula I or a solution of this or these derivatives which additionally contains one or more other polymers and/or physiologically acceptable auxiliaries being spray-dried, or

b) one or more polyamino-dicarboxylic acid-co-imide derivatives of the formula I being dissolved in a solvent or solvent mixture with high melting point, or these derivatives being mixed with one or more other polymers and/or physiologically acceptable auxiliaries and dissolved in a solvent mixture with high melting point and then added dropwise to a condensed cold gas and subsequently the solvent being removed, or

c) one or more polyamino-dicarboxylic acid-co-imide derivatives of the formula I being dissolved in a solvent or solvent mixture and subsequently, where appropriate after addition of another solvent and/or of one or more other polymers, being precipitated or dispersed in water, and the resulting suspension being freed of solvents.

**6.** The use of ultrasonic contrast agents as claimed in one or more of claims 1 to 4 for preparing diagnostic or therapeutic agents.

**7.** The use of ultrasonic contrast agents as claimed in one or more of claims 1 to 4 for investigating cavities in humans, animals or plants.

**8.** The use of ultrasonic contrast agents as claimed in one or more of claims 1 to 4 for diagnosing cardiovascular disorders.

**9.** A diagnostic or therapeutic agent composed of at least one ultrasonic contrast agent as claimed in one or more of claims 1 to 4 or of at least one ultrasonic contrast agent prepared by process 5.

**10.** A process for the preparation of a diagnostic or therapeutic agent as claimed in claim 9, which comprises converting at least one ultrasonic contrast agent as claimed in one or more of claims 1 to 4 with a physiological vehicle and, where appropriate, further additives and/or auxiliaries into a suitable administration form.

**Claims for the following Contracting State : ES**

**1.** A process for the preparation of an ultrasonic contrast agent composed of microparticles which contain

a gas and a polyamino-dicarboxylic acid-co- imide derivative of the formula I,

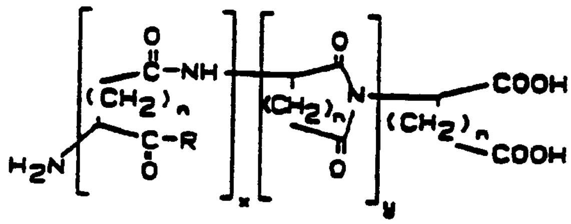

(I)

in which

| | | |
|---|---|---|
| n | is 1 or 2 | |
| x | is 1 to 500 | |
| y | is 1 to 500, where | |
| x + y | is 2 to 1000, and | |
| R | is O-$R^1$ or NH-$R^2$, in which | |
| | $R^2$ | is H, $(CH_2)_m$-O$R^1$, $(CH_2)_m$-O-C(O)-$R^1$ or $(CH_2)_m$-O-C(O)-O$R^1$, and |
| | m | is 2 to 6, and |
| | $R^1$ | is H, aryl, aralkyl, arylalkenyl, alkyl or $C_3$-$C_8$-cycloalkyl or a biologically inactive steroid alcohol or an amino acid, where aryl is unsubstituted or is substituted by $C_1$-$C_4$-alkyl, $C_2$-$C_4$-alkenyl, $C_1$-$C_4$-alkylcarbonyloxy, $C_1$-$C_4$-alkoxycarbonyl, $C_1$-$C_4$-alkoxy or hydroxyl, |

where the alkyl radicals specified for $R^1$ have 1-22 carbon atoms and the alkenyl radicals have 2-22 carbon atoms, which are not interrupted or are interrupted by a carbonyloxy or oxycarbonyl group, where the repeating units placed in square brackets are distributed randomly and/or in blocks in the polymer, and where both the repeating units labeled with x and those labeled with y are identical or different and where the amino acids are α- and/or β-linked , which comprises

a) a solution of one or more polyamino-dicarboxylic acid-co-imide derivatives of the formula I or a solution of this or these derivatives which additionally contains one or more other polymers and/or physiologically acceptable auxiliaries being spray-dried, or

b) one or more polyamino-dicarboxylic acid-co-imide derivatives of the formula I being dissolved in a solvent or solvent mixture with high melting point, or these derivatives being mixed with one or more other polymers and/or physiologically acceptable auxiliaries and dissolved in a solvent mixture with high melting point and then added dropwise to a condensed cold gas and subsequently the solvent being removed, or

c) one or more polyamino-dicarboxylic acid-co-imide derivatives of the formula I being dissolved in a solvent or solvent mixture and subsequently, where appropriate after addition of another solvent and/or of one or more other polymers, being precipitated or dispersed in water, and the resulting suspension being freed of solvents.

2. The process as claimed in claim 1, which comprises preparing compounds of the formula I in which

| | |
|---|---|
| R | is NH-$R^2$ and |
| m | is 2 and |
| $R^1$ | is H, aryl, aralkyl, alkyl or $C_5$-$C_6$-cycloalkyl, where the alkyl radicals have 1-22 carbon atoms. |

3. The process as claimed in claim 1 or 2, which comprises preparing compounds of the formula I in which

| | |
|---|---|
| R | is O-$R^1$ and |
| $R^1$ | is aryl, aralkyl, alkyl or $C_5$-$C_6$-cycloalkyl, where the alkyl radicals have 1-22 carbon atoms. |

4. The process as claimed in one or more of claims 1 to 3, which comprises preparing microparticles which contain air, nitrogen, noble gases, hydrogen, carbon dioxide, oxygen or mixtures of these gases as gas.

5. The use of ultrasonic contrast agents obtained as claimed in one or more of claims 1 to 4 for preparing diagnostic or therapeutic agents.

6. The use of ultrasonic contrast agents obtained as claimed in one or more of claims 1 to 4 for preparing diagnostic agents for investigating cavities in humans, animals or plants.

7. The use of ultrasonic contrast agents as claimed in one or more of claims 1 to 4 for preparing diagnostic agents for diagnosing cardio-vascular disorders.

8. A process for the preparation of a diagnostic or therapeutic agent, which comprises converting at least one ultrasonic contrast agent obtained as claimed in one or more of claims 1 to 4 with a physiological vehicle and, where appropriate, further additives and/or auxiliaries into a suitable administration form.

## Revendications

**Revendications pour les Etats contractants suivants : AT, BE, CH, DE, DK, FR, GB, IT, LI, NL**

1. Agents de contraste ultrasonique constitués de microparticules qui contiennent un gaz et un dérivé de poly(acide aminodicarboxylique)-co-imide de formule I

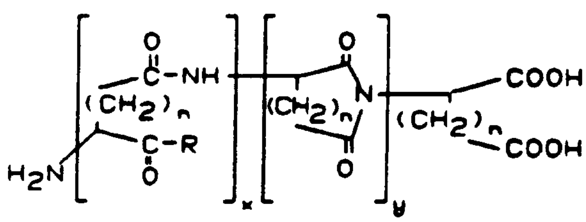

(I)

dans laquelle

n est 1 ou 2,
x va de 1 à 500,
y va de 1 à 500,
x + y allant de 2 à 1 000, et
R représente O-$R^1$ ou NH-$R^2$, groupes dans lesquels
$R^2$ représente H, $(CH_2)_m$-O$R^1$, $(CH_2)_m$-O-C(O)-$R^1$ ou $(CH_2)_m$-O-C(O)-O$R^1$, et
m va de 2 à 6, et
$R^1$ représente H ou un radical aryle, aralkyle, arylalcényle, alkyle ou cycloalkyle en $C_3$-$C_8$, ou un alcool de type stéroïde biologiquement inactif ou un amino-acide, le radical aryle étant non substitué ou étant substitué par un groupe alkyle en $C_1$-$C_4$, alcényle en $C_2$-$C_4$, alkyl($C_1$-$C_4$)-carbonyloxy, alcoxy($C_1$-$C_4$)-carbonyle, alcoxy en $C_1$- $C_4$ ou hydroxy,

les radicaux alkyle mentionnés pour $R^1$ comportant de 1 à 22 atomes de carbone et les radicaux alcényle comportant de 2 à 22 atomes de carbone, qui ne sont pas interrompus ou sont interrompus par un groupe carbonyloxy ou oxycarbonyle,
les motifs répétitifs mis entre crochets étant répartis statistiquement et/ou en séquences dans le polymère, et aussi bien les motifs répétitifs caractérisés par x que ceux caractérisés par y étant identiques ou différents, et les aminoacides étant liés en $\alpha$ et/ou $\beta$.

2. Agents de contraste ultrasonique selon la revendication 1, dans lesquels, dans la formule I,
R représente NH-$R^2$ et
m est 2, et
$R^1$ représente H ou un groupe aryle, aralkyle, alkyle ou cycloalkyle en $C_5$-$C_6$, les radicaux alkyle ayant de 1 à 22 atomes de carbone.

3. Agents de contraste ultrasonique selon la revendication 1, dans lesquels, dans la formule I,
R représente O-$R^1$ et
$R^1$ représente un groupe aryle, aralkyle, alkyle ou cycloalkyle en $C_5$-$C_6$, les radicaux alkyle comportant de 1 à 22 atomes de carbone.

4. Agents de contraste ultrasonique selon une ou plusieurs des revendications 1 à 3, caractérisés en ce que les microparticules contiennent en tant que gaz de l'air, de l'azote, des gaz rares, de l'hydrogène, du dioxyde de carbone, de l'oxygène ou des mélanges de ces gaz.

5. Procédé pour la préparation d'un agent de contraste ultrasonique selon la revendication 1, caractérisé en ce que
   a) on sèche par atomisation une solution d'un ou plusieurs dérivés de poly(acide aminodicarboxylique)-co-imides de formule I ou une solution de celui-ci ou de ses dérivés, qui contient en outre un ou plusieurs autres polymères et/ou adjuvants physiologiquement acceptables, ou en ce que
   b) on dissout un ou plusieurs dérivés de poly(acide aminodicarboxylique)-co-imides de formule I dans un solvant ou mélange de solvants à haut point de fusion, ou on mélange ces dérivés avec un ou plusieurs autres polymères et/ou adjuvants physiologiquement acceptables, et on les dissout dans un mélange de solvants à haut point de fusion, et on les introduit ensuite goutte à goutte dans un gaz froid condensé, puis on élimine le solvant, ou en ce que
   c) on dissout un ou plusieurs dérivés de poly(acide aminodicarboxylique)-co-imides de formule I dans un solvant ou mélange de solvants et ensuite on les disperse dans l'eau ou on les fait précipiter, éventuellement après addition d'un autre solvant et/ou d'un ou plusieurs autres polymères, et on sépare les solvants de la suspension obtenue.

6. Utilisation d'agents de contraste ultrasonique selon une ou plusieurs des revendications 1 à 4, pour la préparation d'agents de diagnostic ou d'agents thérapeutiques.

7. Utilisation d'agents de contraste ultrasonique selon une ou plusieurs des revendications 1 à 4, pour l'examen de cavités chez l'homme, l'animal ou des végétaux.

8. Utilisation d'agents de contraste ultrasonique selon une ou plusieurs des revendications 1 à 4, pour le diagnostic de maladies cardiovasculaires.

9. Agent de diagnostic ou agent thérapeutique, constitué d'au moins un agent de contraste ultrasonique selon une ou plusieurs des revendications 1 à 4, ou d'au moins un agent de contraste ultrasonique préparé selon le procédé 5.

10. Procédé pour la préparation d'un agent de diagnostic ou d'un agent thérapeutique selon la revendication 9, caractérisé en ce que l'on met sous une forme d'administration appropriée au moins un agent de contraste ultrasonique selon une ou plusieurs des revendications 1 à 4, avec un véhicule physiologique et éventuellement d'autres adjuvants et/ou additifs.

**Revendications pour l'Etat contractant suivant : ES**

1. Procédé pour la préparation d'un agent de contraste ultrasonique constitué de microparticules qui contiennent un gaz et un dérivé de poly(acide aminodicarboxylique)-co-imide de formule I

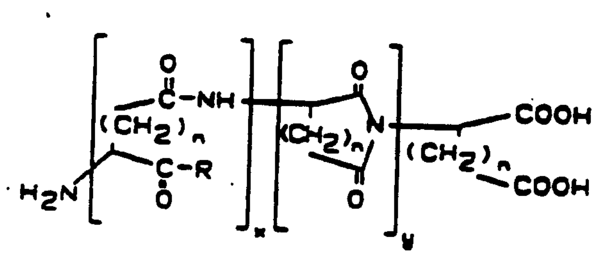

(I)

dans laquelle

| | | | |
|---|---|---|---|
| n | est 1 ou 2, | | |
| x | va de 1 à 500, | | |
| y | va de 1 à 500, | | |
| x + y | allant de 2 à 1 000, et | | |
| R | représente 0-$R^1$ ou NH-$R^2$, groupes dans lesquels | | |
| | | $R^2$ | représente H, $(CH_2)_m$-O$R^1$ , $(CH_2)_m$-O-C(O)-$R^1$ ou $(CH_2)_m$-O-C(O)-O$R^1$, et |
| | | m | va de 2 à 6, et |
| | | $R^1$ | représente H ou un radical aryle, aralkyle, arylalcényle, alkyle ou cycloalkyle en $C_3$-$C_8$, ou un alcool de type stéroïde biologiquement inactif ou un aminoacide, le radical |

aryle étant non substitué ou étant substitué par un groupe alkyle en $C_1$-$C_4$, alcényle en $C_2$-$C_4$, alkyl($C_1$-$C_4$)-carbonyloxy, alcoxy($C_1$-$C_4$)-carbonyle, alcoxy en $C_1$-$C_4$ ou hydroxy,

les radicaux alkyle mentionnés pour $R^1$ comportant de 1 à 22 atomes de carbone et les radicaux alcényle comportant de 2 à 22 atomes de carbone, qui ne sont pas interrompus ou sont interrompus par un groupe carbonyloxy ou oxycarbonyle,

les motifs répétitifs mis entre crochets étant répartis statistiquement et/ou en séquences dans le polymère, et aussi bien les motifs répétitifs caractérisés par x que ceux caractérisés par y étant identiques ou différents, et les aminoacides étant liés en $\alpha$ et/ou $\beta$,

caractérisé en ce que

a) on sèche par atomisation une solution d'un ou plusieurs dérivés de poly(acide aminodicarboxylique)-co-imides de formule I ou une solution de celui-ci ou de ses dérivés, qui contient en outre un ou plusieurs autres polymères et/ou adjuvants physiologiquement acceptables, ou en ce que

b) on dissout un ou plusieurs dérivés de poly(acide aminodicarboxylique)-co-imides de formule I dans un solvant ou mélange de solvants à haut point de fusion, ou on mélange ces dérivés avec un ou plusieurs autres polymères et/ou adjuvants physiologiquement acceptables, et on les dissout dans un mélange de solvants à haut point de fusion, et on les introduit ensuite goutte à goutte dans un gaz froid condensé, puis on élimine le solvant, ou en ce que

c) on dissout un ou plusieurs dérivés de poly(acide aminodicarboxylique)-co-imidesde formule I dans un solvant ou mélange de solvants et ensuite on les disperse dans l'eau ou les fait précipiter, éventuellement après addition d'un autre solvant et/ou d'un ou plusieurs autres polymères, et on sépare les solvants de la suspension obtenue.

**2.** Procédé selon la revendication 1, caractérisé en ce que l'on prépare des composés de formule dans lesquels

| | |
|---|---|
| R | représente NH-$R^2$ et |
| m | est 2, et |
| $R^1$ | représente H ou un groupe aryle, aralkyle, alkyle ou cycloalkyle en $C_5$-$C_6$, les radicaux alkyle ayant de 1 à 22 atomes de carbone. |

**3.** Procédé selon la revendication 1 ou 2, caractérisé en ce que l'on prépare des composés de formule I dans lesquels

| | |
|---|---|
| R | représente O-$R^1$ et |
| $R^1$ | représente un groupe aryle, aralkyle, alkyle ou cycloalkyle en $C_5$-$C_6$, les radicaux alkyle comportant de 1 à 22 atomes de carbone. |

**4.** Procédé selon une ou plusieurs des revendications 1 à 3, caractérisé en ce que l'on prépare des microparticules qui contiennent en tant que gaz de l'air, de l'azote, des gaz rares, de l'hydrogène, du dioxyde de carbone, de l'oxygène ou des mélanges de ces gaz.

**5.** Utilisation d'agents de contraste ultrasonique obtenus selon une ou plusieurs des revendications 1 à 4, pour la préparation d'agents de diagnostic ou d'agents thérapeutiques.

**6.** Utilisation d'agents de contraste ultrasonique obtenus selon une ou plusieurs des revendications 1 à 4, pour l'examen de cavités chez l'homme, l'animal ou des végétaux.

**7.** Utilisation d'agents de contraste ultrasonique, selon une ou plusieurs des revendications 1 à 4, pour la préparation d'agents de diagnostic pour le diagnostic de maladies cardiovasculaires.

**8.** Procédé pour la préparation d'un agent de diagnostic ou d'un agent thérapeutique, caractérisé en ce que l'on met sous une forme d'administration appropriée au moins un agent de contraste ultrasonique obtenu selon une ou plusieurs des revendications 1 à 4, avec un véhicule physiologique et éventuellement d'autres adjuvants et/ou additifs.